# EUROPÄISCHE PATENTSCHRIFT

(11) **EP 0 776 363 B1**
(45) Veröffentlichungstag und Bekanntmachung des Hinweises auf die Patenterteilung: **22.12.1999**
(21) Anmeldenummer: 95918531.5
(22) Anmeldetag: 19.05.1995
(51) Int. Cl.: C12N 15/11, C12N 15/63, C12N 15/86, C12N 9/00

(54) **RIBOZYM-BIBLIOTHEK, IHRE HERSTELLUNG UND IHRE VERWENDUNG**
RIBOZYME LIBRARY, ITS PRODUCTION AND USE
BIBLIOTHEQUE DE RIBOZYMES, SA PRODUCTION ET SON UTILISATION

(30) Priorität: 04.07.1994 DE 4424762
(43) Veröffentlichungstag der Anmeldung: 04.06.1997
(73) Patentinhaber: Max-Planck-Gesellschaft zur Förderung der Wissenschaften e.V., 80539 München (DE)
(72) Erfinder: LIEBER, Andre, D-13189 Berlin (DE); STRAUSS, Michael, D-13187 Berlin (DE)
(74) Vertreter: Baumbach, Friedrich
(86) Internationale Anmeldenummer: DE9500662
(87) Internationale Veröffentlichungsnummer: WO9601314

(56) Entgegenhaltungen:
- FR-A- 2 687 411
- J. CELL BIOCHEM SUPPL. 0, Bd. 17, Nr. E, 1993 MACEJAK, D.G. AND DRAPER, K. 'Design of quasi-random ribozyme expression vectors'

## Beschreibung

Die Erfindung betrifft eine Ribozym-Bibliothek, ihre Herstellung und Verwendung. Anwendungsgebiete der Erfindung sind die Molekularbiologie, die Gentechnik und die Medizin.

Die Inaktivierung von Genfunktionen durch reverses genetisches Material ist die wichtigste Methode, um bestimmte Gene abzuschalten. Das ist von großer Bedeutung zur Bekämpfung von infektiösen und anderen, durch Störung der Genexpression bedingten Krankheiten(einschließlich AIDS). Eine Genfunktion kann in verschiedenen Ebenen außer Kraft gesetzt werden: durch homologe Rekombination auf der DNA-Ebene, durch Antisense-Nukleinsäuren oder Ribozyme auf der RNA-Ebene oder durch Antikörper auf der Proteinebene. In der praktischen Umsetzung haben alle 4 Möglichkeiten Vor- und Nachteile. Für eine therapeutische Anwendung scheint nur die RNA-Inaktivierung durch Antisense-Moleküle oder durch Ribozyme durchführbar zu sein. Beide Verbindungsklassen können durch chemische Synthese oder in Verbindung mit einem Promoter durch biologische Expression in vitro oder sogar in vivo hergestellt werden. Das Prinzip der katalytischen Selbstspaltung von RNA-Molekülen und der Spaltung in trans hat sich in den letzten 10 Jahren gut etabliert. Innerhalb der RNA-Moleküle mit Ribozym-Aktivität sind die Hammerhead-Ribozyme am besten charakterisiert. Nachdem gezeigt worden ist, daß Hammerhead-Strukturen in heterologe RNA-Sequenzen integriert werden und dadurch die Ribozym-Aktivität auf dieses Molekül übertragen können, scheint es naheliegend, daß katalytische Antisense-Sequenzen für fast jede Zielsequenz mit einem übereinstimmenden Spaltort vorgesehen werden können.

Das Grundprinzip der Ribozym-Ausstattung ist sehr einfach: Man wählt eine interessierende Region der RNA aus, die das Triplett GUC (bzw. CUC) enthält, nimmt 2 Oligonukleotid-Stränge mit je 6-8 Nukleotiden und fügt die katalytische Hammerhead-Sequenz dazwischen ein.

Moleküle dieser Art wurden für zahlreiche Zielsequenzen synthetisiert, sie zeigten katalytische Aktivität in vitro und in manchen Fällen auch in vivo. Die besten Ergebnisse wurden mit kurzen Ribozymen und Zielsequenzen erzielt. Eine aktuelle Herausforderung für die in vivo-Anwendung ist die Konstruktion von Ribozymgenen, die eine kontinuierliche Expression des Ribozyms in einer bestimmten Zelle erlauben(Bertrand, E. et al. /1994/ Nucleic Acids Res. 22, 293-300).

Es gibt 5 potentielle Gründe, die eine befriedigende Funktion von exprimierten Ribozymen innerhalb des komplexen Zellmilieus behindern.
1. Innerhalb der Zelle existiert das mRNA-Substrat vermutlich in einer stark gefalteten Struktur, die außerdem noch durch an Teile der Struktur gebundene Proteine geschützt sein kann. Das Treffen von zugänglichen Orten innerhalb des Substrates zur Hybridisierung mit den komplementären flankierenden Regionen des Ribozyms ist eine Frage der aktuellen Wahrscheinlichkeit. Computergestützte Vorhersagen von möglichen thermodynamisch stabilen Sekundärstrukturen können für die Suche nach Loop-Regionen ohne Basenpaarung nützlich sein, aber die physiologische Relevanz dieser Konformationsmodelle ist noch unsicher.
2. Da die Ziel-mRNA sofort aus dem Zellkern heraustransportiert wird, muß das Ribozym muß auch in das Zytoplasma übergehen, bevorzugt auf dem selben Wege. Es ist jedoch schwierig, eine Kolokalisation von Ribozymen und ihrem Substrat zu erreichen.
3. Der Einsatz von Ribozymen in vivo erfordert die Einfügung von Ribozymgenen in geeignete Expressionskassetten. Die Transkription dieser Konstrukte kann mRNAs produzieren, in denen die zentrale katalytische Sekundärstruktur der Ribozyme durch andere, stabilere Basenpaarungen innerhalb der nichtkomplementären flankierenden Sequenzen verdrängt wird.
4. Ein Überschuß (100-1000fach) an Ribozym-Molekülen gegenüber der Zielsequenz ist notwendig, um ein registrierbares Ansteigen des RNA-Niveaus zu erreichen. Die Produktion von 10⁵-10⁶ Ribozym-Molekülen pro Zelle über eine lange Periode hinweg kann jedoch zytotoxische Wirkung haben. Im allgemeinen sind solche hohen Expressionsniveaus nicht stabil. Die Notwendigkeit des Überschusses an Ribozymen wird durch die ungenügende Stabilität der Ribozyme gegenüber Nukleasen, durch den uneffektiven Transport zum Zytoplasma und durch den nicht optimalen Umsatz-Faktor der Spaltungsreaktion hervorgerufen.
5. Die Kinetik der Spaltungsreaktion und die Fähigkeit der Ribozyme, Multi-Umsatz-Reaktionen durchzuführen, hängt von den Bindungsparametern und der Struktur der komplementären flankierenden Regionen der Ribozyme ab. Zelluläre Proteine können die Katalyse der Spaltungsreaktion beeinflussen, wahrscheinlich mit Hilfe der Dissoziation des Ribozyms vom Substrat der Spaltung, das die Vorstufe zur nächsten Spaltung darstellt. Bis heute ist es nicht möglich, die optimale Struktur der flankierenden Regionen für ein Ribozym vorherzusagen, um hohe Spezifität und einen hohen Umsatz zu garantieren.
   In J. Cell Biochem Suppl. 0, Vol. 17, No. E (1993) wurde von Macejak, DG und Draper, K die Inkubation eines die gewünschte Zielsequenz enthaltenden Materials mit einer Ribozym-Bibliothek beschrieben sowie die Identifizierungsmöglichkeit erhaltener Spaltprodukte und die Isolierung der für die Spaltung verantwortlichen Ribozyme.
   Insgesamt kann man feststellen, daß trotz vieler Bemühungen zur Konstruktion spezifischer Ribozym-Gene nur Teilerfolge erzielt wurden, meist auf der Basis von "trial and error"-Experimenten.

Die Erfindung hat das Ziel, die Bereitstellung optimaler Ribozyme für beliebige Zielsequenzen zu ermöglichen. Diese Ribozyme sollen effektiv exprimierbar, stabil und in vivo einsetzbar sein, vorzugsweise für die Identifizierung und zum Abschalten von Genen bei Erkrankungen.

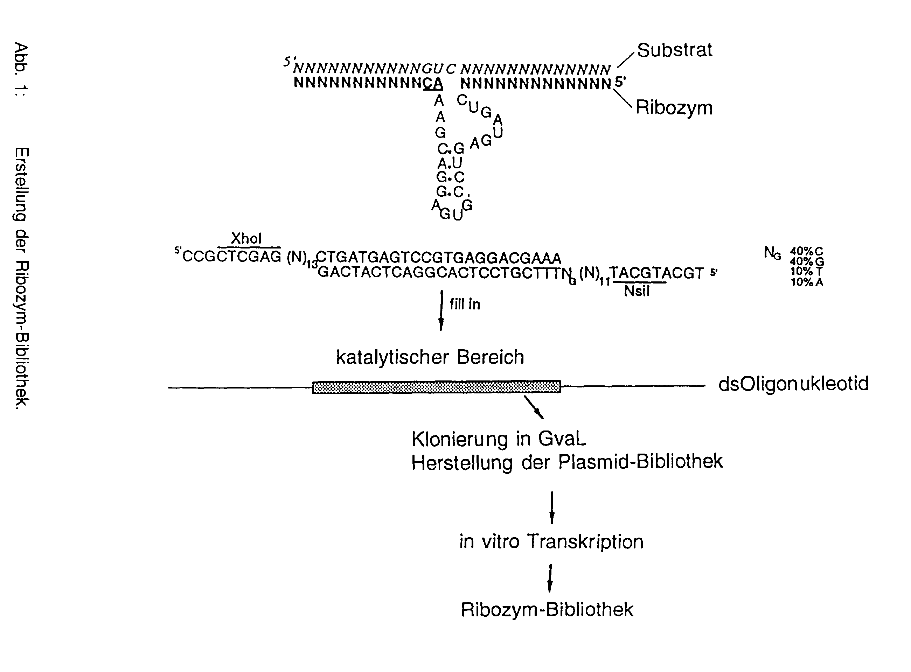

Der Grundgedanke der Erfindung besteht darin, das Verfahren so zu führen, daß eine gesuchte bzw. für das Abschalten vorgesehene Zielsequenz sich das passendste Ribozym aus einem Angebot von Ribozymen mit bekannter Stabilität und Struktur selbst heraussucht. Das wird erfindungsgemäß dadurch realisiert, daß mittels einer optimierten Expressionskassette eine Ribozym-Bibliothek angelegt wird, welche 10⁹ bis 10¹¹ Ribozymgene enthält. Die erfindungsgemäß optimierte Expressionskassette besteht aus einem adenoviralen va-RNA-Gen und einer stabilen Loop-Region mit den Ribozymgenen innerhalb der Loop-Region. Die Ribozyme setzen sich aus einer zentralen Hammerhead-Struktur definierter Sequenz und flankierenden Sequenzen mit je 6-13 Nukleotiden Länge zufälliger Basenfolge zusammen. Die Hammerhead-Struktur wird durch ein doppelsträngiges Gen kodiert, in welchem das Hammerhead auf beiden Seiten von den flankierenden Sequenzen zufälliger Basenfolge eingeschlossen ist.
Die doppelsträngige Hammerhead-Region hat bevorzugt folgende Sequenz: CTGATGAGTCCGTGAGGACGAAAC.
Der Aufbau der erfindungsgemäßen Ribozym-Bibliothek geht von synthetischen Oligonukleotiden mit einer Zufallssequenz von 6-13 Nukleotiden aus. Diese werden mit der Ribozymsequenz verbunden, in einen Doppelstrang umgewandelt und über flankierende Restriktionsorte in die entsprechende Insertionsstelle der Expressionskassette kloniert. In Abbildung 1 ist der Aufbau der Ribozym-Bibliothek schematisch dargestellt.

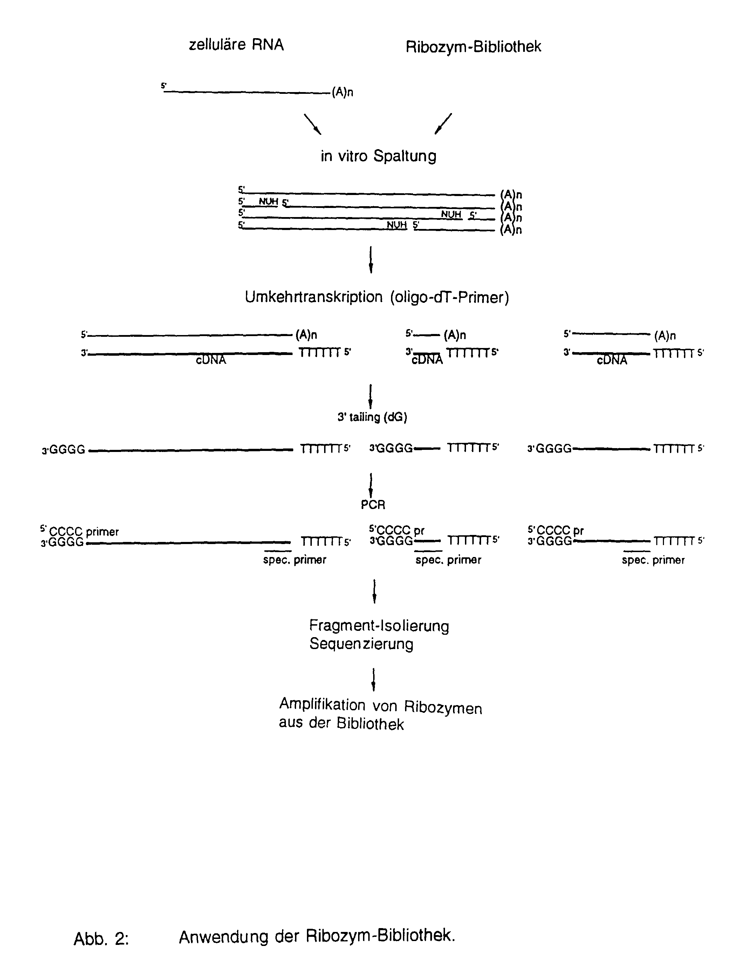

Abbildung 2 zeigt schematisch die Anwendung der Ribozym-Bibliothek; sie wird nachfolgend am Beispiel des Wachstumshormons erläutert. Wachstumshormon hat 150 theoretische Spaltstellen (Ribozym-Bindungsorte, Basenfolgen GUC bzw. CUC). Nur einige Spaltstellen werden in vivo zugänglich sein. Diese und die dazu passenden effektivsten Ribozyme sollen isoliert werden.
Dazu wird zunächst ein Pool von Ribozym-Genen synthetisch hergestellt, wobei eine zentrale "hammerhead" Ribozymsequenz von je 13 Nukleotiden zufälliger Reihenfolge flankiert wird. Diese Gene werden in einen Expressionsvektor (GvaL) für Ribozyme kloniert, wobei sie auf beiden Seiten von einer identischen Sequenz von 21 Nukleotiden flankiert werden, die eine Sekundärstrukturbildung verhindern sollen. Durch die Klonierung entsteht eine Bibliothek von ca. 10⁹ Klonen. Zur Isolierung eines spezifischen Ribozyms wird das Zielgen in vitro von einem geeigneten Konstrukt transkribiert (mit T7-Polymerase oder RNA-Polymerase III) und die erhaltene RNA mit der ebenfalls in vitro transkribierten Ribozym-Bibliothek inkubiert. Anschließend werden die Spaltprodukte elektrophoretisch getrennt. Deutlich erkennbare Fragmente werden aus dem Gel präpariert und sequenziert.
Die Sequenz an den Enden der Fragmente erlaubt die Festlegung der Spaltstelle und Identifizierung des für die Spaltstelle verantwortlichen Ribozyms. Das betreffende Ribozym wird unter Verwendung von zwei für seine flankierenden Sequenzen spezifischen Oligonukleotiden aus der Ribozym-Bibliothek amplifiziert und erneut in den Vektor GvaL kloniert (Beispiel 1). Die Ribozymaktivität der Bibliothek wird durch Inkubation mit zellulärer RNA und deren Degradation nachgewiesen (Beispiel 2). Das Vorhandensein von Ribozymen gegen eine bestimmte Ziel-RNA, z.B. hGH, wird durch Inkubation mit einer in vitro transkribierten RNA nachgewiesen (Beispiel 3). Die Lokalisierung der Spaltstellen erfolgt durch Isolierung von Fragmenten der gespaltenen Ziel-RNA und deren Sequenzierung (Beispiel 4). Die Spezifität und Effektivität der aus der Bank isolierten und reklonierten Ribozyme wird durch deren Inkubation mit der Ziel-RNA bestimmt (Beispiel 5).
Die biologische Wirksamkeit, d.h. die Ausschaltung der Funktion der Ziel-RNA in vivo, wird durch Transfektion des reklonierten Ribozyms mit dem Zielgen in geeignete Zellen (z.B. CHO) und nachfolgende Bestimmung der spezifischen Proteinsynthese (z.B. hGH-Sekretion) ermittelt (Beispiel 6).

Die Erfindung soll nachfolgend im einzelnen durch Ausführungsbeispiele näher erläutert werden.

### Beispiel 1:

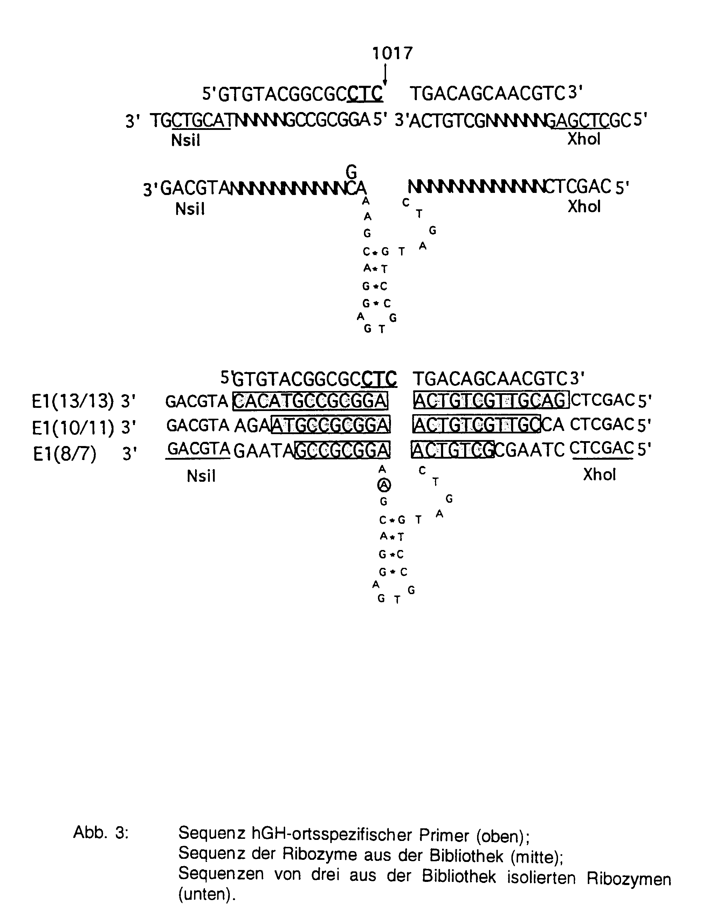

Die Strategie ist schematisch in Abb. 3 dargestellt. Der mittlere Teil der Abbildung zeigt die Struktur des vorgesehenen Ribozympools, der obere die aktuellen Sequenzen der zwei Oligonukleotide, die angelagert und zur Bildung von doppelsträngigen Ribozym-Genen aus der Bank verlängert werden. Das entstehende Fragmentgemisch wurde in die Gval-Kassette als Xhol-Nsil-Fragment einkloniert. Eine Bibliothek von 10⁹ verschiedenen Varianten wurde geschaffen. Die Ribozyme wurden in vitro entweder durch T7-Polymerase oder polIII von einem HeLa-Extrakt synthetisiert. Als Zielsequenz wurde RNA von CHO-Zellen benutzt, welche das hGH-Gen stabil exprimiert (5000 RNA-Kopien/Zelle). Gereinigte RNA wurde mit der in vitro synthetisierten Ribozym-Bibliothek inkubiert. Nach der Reinigung der Spaltprodukte an einer oligo-dT-Säule wurde das 5'-Ende der stromabwärts-Spaltprodukte mittels RACE-Technik wie folgt analysiert: Nach der reversen Transkription mit oligo-dT-Primern werden die cDNAs am 3'-Ende mit dG verbunden, mit einem oligodC amplifiziert und mit hGH-spezifischen Primern behandelt, in pGEMT (Promega) einkloniert und sequenziert. Die Sequenzen sollten unmittelbar stromabwärts von der NUH-Erkennungsseite (GUC, CUC) innerhalb der hGH-RNA starten. Das Gen für das Ribozym, das die Spaltung eines ausgewählten Ortes bewirkte, wurde durch PCR aus der Ribozym-Plasmid-Bibliothek amplifiziert, wobei spezifische degenerierte Primer für die flankierenden Regionen der Ribozym-Gene verwendet wurden. Nach Amplifikation wurde das entstandene Fragment zwischen die PstI- und SalI-Orte des Vektors GvaL kloniert. Unter den sequenzierten 50 Klonen wurden für drei Ribozym-Spaltorte Ribozyme mit unterschiedlich langen Flanken (7-13 Nukleotide) gefunden(Abb. 3, unten).

### Beispiel 2:

Die Spaltung von zellulärer RNA wurde bei physiologischem pH (50mM Tris-Cl, pH 7,5) bei 37°C innerhalb einer Stunde mit oder ohne vorhergehende Hitzedenaturierung (90 sec bei 95°C) in einem 15 µl Reaktionsgefäß durchgeführt. Folgende Ansätze wurden gewählt:
1./2. Gereinigte Total-RNA (1µg/Probe) als Ziel. Ribozyme GvaLRz als T7-Transkript, in vitro-Transkript von GvaL diente als Kontrolle.
3. Zytoplasmatische RNA/Protein-Fraktion als Ziel. 10⁵ Zellen wurden im 50mM Tris-HCL (pH 7,5) 10 min in Eis lysiert, in flüssigem Stickstoff eingefroren, aufgetaut bei 37°C, und die Kerne wurden durch Zentrifugationn entfernt. Als Ribozym wurde ein T7-Transkript von GvaLRz (10µg) verwendet.
4. Zytoplasmatische RNA als Ziel. Das Ribozym wurde durch pol III-Transkription (2µg/Probe) hergestellt.
Die deutlichsten Spaltungen werden mit T7-Transkripten und totaler bzw. auch zytoplasmatischer RNA erhalten.

### Beispiel 3:

Spezifische in vitro-Spaltung von hGH-mRNA durch Ribozym aus der Bibliothek.
Totale oder zytoplasmatische RNA-Präparationen aus hGH-produzierenden Zellen werden mit Ribozymen aus der Bibliothek inkubiert, die entweder mit pol III oder T7-Polymerase transkribiert wurde. hGH-spezifische 3'-Fragmente werden revers transkribiert und durch PCR amplifiziert. Die PCR-Bedingungen werden so gewählt, daß hauptsächlich Fragmente >1000bp entstehen. PCR-Produkte werden auf einem 6% PAA-Gel aufgetrennt, Marker für die Fragmentgröße werden auf der linken Bahn aufgetragen. Es wurden 6 spezifische Banden gefunden, deren korrespondierende Fragmentlänge mit einer in 4 Exonorten (E1-E4) und 2 Intronorten (I1,I2) korrelieren. Es ist festzustellen, daß T7 pol Transkripte leichter nachzuweisen sind und daß es mehr Spaltstellen in Total-RNA gibt als in zytoplasmatischer RNA.

### Beispiel 4: Lokalisierung von Ribozym-Spaltorten innerhalb von hGH mRNA

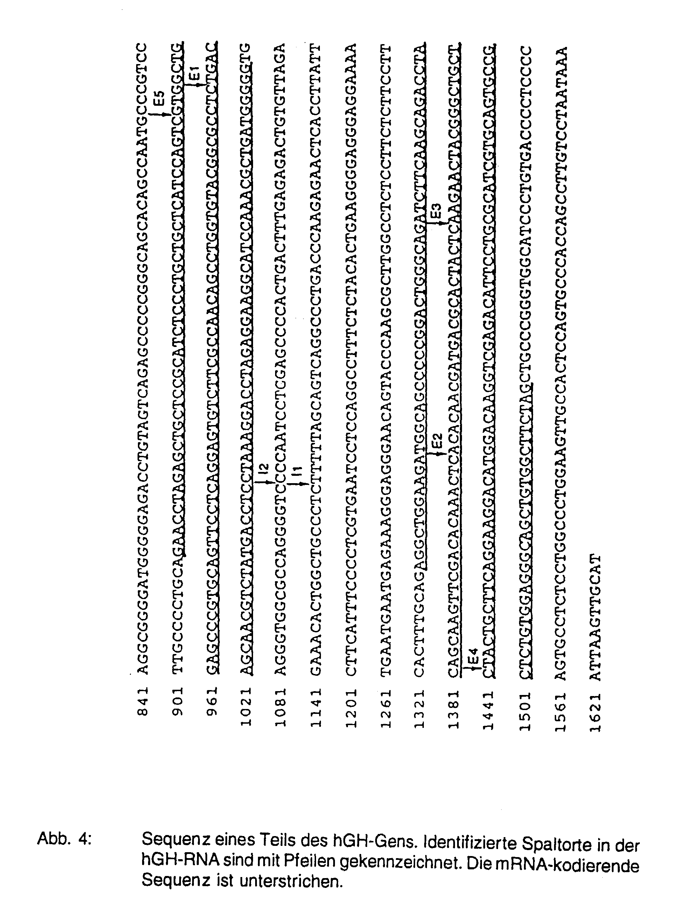

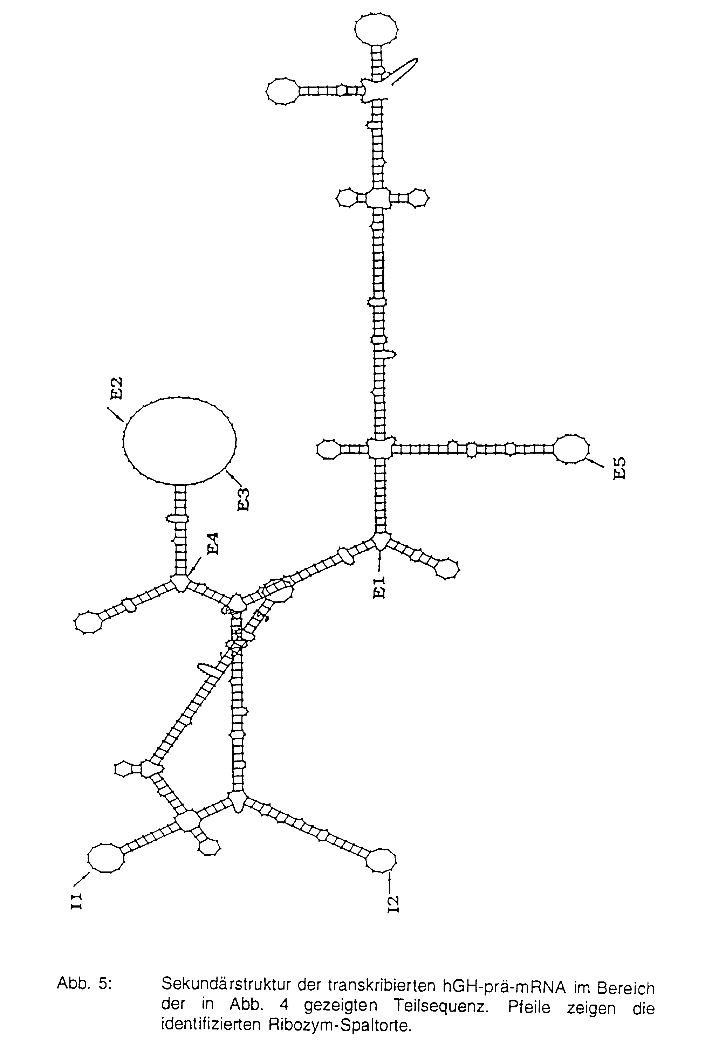

Die Fragmente E1-E4 und I1,I2 werden aus dem Gel geschnitten, gereinigt und in pGEMT einkloniert. Von jedem Fragment werden 20 Klone sequenziert. Etwa 50% der Klone starten entweder an einem CUC- oder einem GUC-Ort. Die anderen 50% repräsentieren wahrscheinlich Abbauprodukte.
Die Spaltorte gemäß den Fragmenten sind in der Sequenz von hGH enthalten.El: 1017 (Exon IV), E2: CTC 1401 (Exon V), E3: CTC 1422 (Exon V), E4: CTC 1441 (Exon V), E5 (stammt von einem gesonderten Experiment): GTC (Exon IV), 12: CTC 1099 (Intron IV), I1: GTC (Intron IV)(Abb.4).
B, Mit dem Programm HUSAR, MFOLD, wurde eine graphische Darstellung mit PLOTFOLD erhalten (Abb.5).

### Beispiel 5: Spaltung in vitro von hGH-spezifischer RNA durch Ribozyme aus der Bibliothek

A. Spaltung mit 3 verschiedenen selektierten Ribozymen. Die Ribozyme werden mit T7-Polymerase von jeweils einem selektierten Klon umgeschrieben und mit in vitro transkribierter hGH RNA der gleichen Molarität (beide 100nM) für 20 min bei 37°C ohne Hitzedenaturierung inkubiert. Proben werden auf ein denaturiertes 6% PAA-Gel aufgebracht. Die entstehenden Bruchstücke haben die erwartete Größe (El: 1017, 646; I1: 1099, 564; E5: 952,711).
B. Spaltung von hGH RNA durch E1-Ribozym mit verschiedener Länge der komplementären Regionen. Die Inkubation erfolgte wie in A. Die Fragmentabtrennung erfolgte auf einem 4% denaturiertem PAA-Gel. Die Länge der komplementären Region des El-Ribozyms beträgt 26=13/13, 21=10/11 bzw. 15=8/7 (Abb.3, unten). Es ist festzustellen, daß die 2 spezifischen Spaltprodukte nur nach Inkubation mit Ribozymen in der Gegenwart von Magnesium nachweisbar sind. Die effektivste Spaltung ist bei der kürzesten Komplementarität zu finden (15=8/7).

### Beispiel 6: Effekt der Ribozymexpression in vivo auf das Niveau der hGH-Sekretion

Die Transfektion von CHO-Zellen erfolgte gleichzeitig mit pCMVhGH, Ribozymen oder Kontrollkonstrukten und pSV2neo. Die Kurzzeit-Expression wurde nach 3 Tagen und die stabile Expression nach Selektion mit Geneticin nach 4 Wochen getestet. Das hGH-Niveau wurde mit ELISA (Nachweisgrenze 3ng/ml) festgestellt. Das Niveau des erhaltenen hGH mit pCMVhGH +GvaL wurde als 100% angesetzt(Kurzzeit: 7 µghGH/ml/24hrs; stabile: 2µg hGH/ml/24hrs).
Die Resultate eines typischen Experiments sind in Tabelle 1 zusammengestellt.

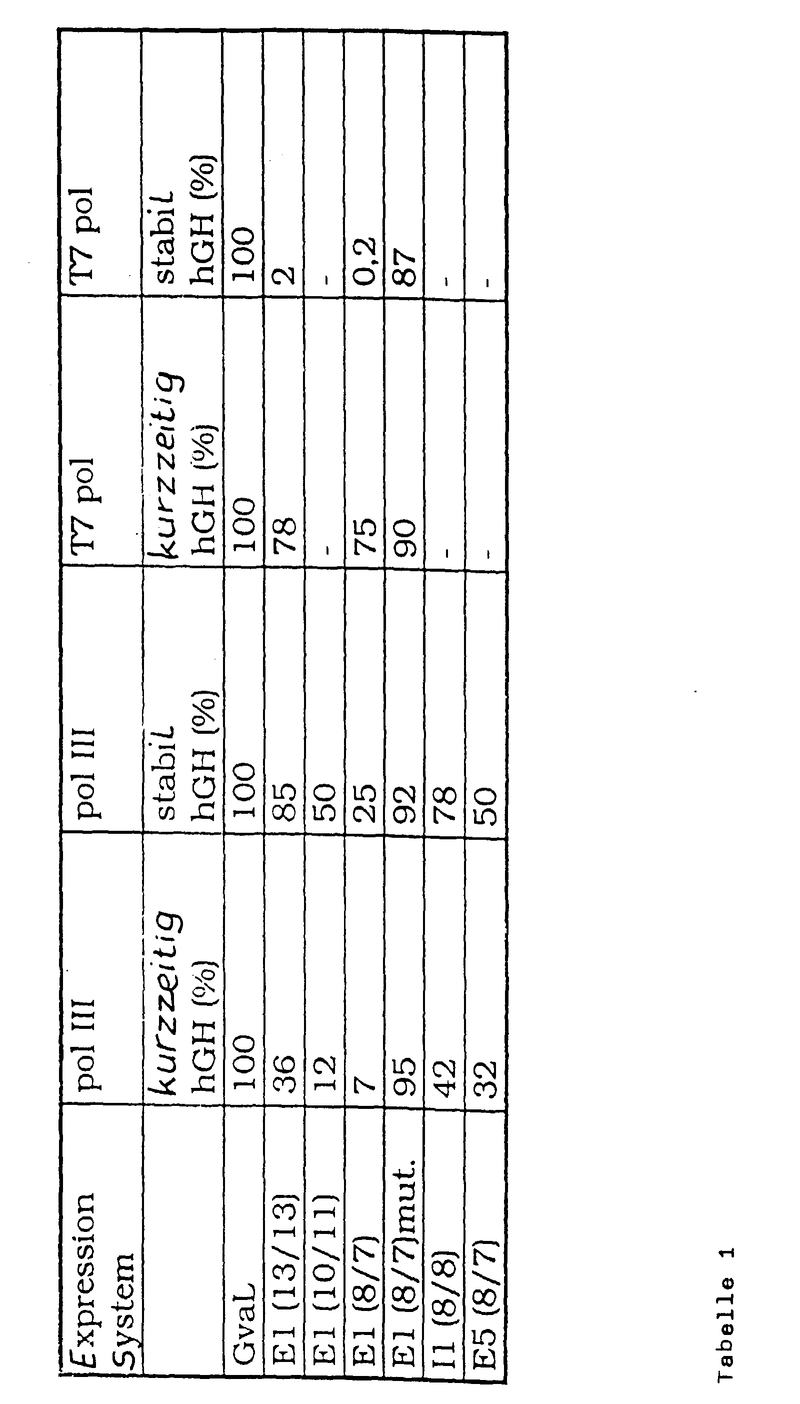

## Patentansprüche

1. Expressionskassette bestehend aus einem T7-Promoter, einem adenoviralen va-RNA-Gen und einer stabile Loop-Region mit einem Ribozym-Gen innerhalb der Loop-Region, wobei das Ribozym-Gene aus einer zentralen Hammerhead-Struktur, definierter Sequenz und flankierenden Sequenzen auf beiden Seiten der Hammerhead-Struktur mit je 6-13 Nukleotiden zufälliger Basenfolge besteht.

2. Expressionskassette nach Anspruch 1,
dadurch gekennzeichnet,
daß die zentrale Hammerhead-Struktur eine doppelsträngige DNA der Sequenz CTGATGAGTCCGTGAGGACGAAAC umfaßt.

3. Verwendung der Expressionskassette gemäß Anspruch 1 oder 2 zum Aufbau einer Ribozym-Bibliothek mit 10⁹ - 10¹¹ Ribozym-Genen.

4. Verfahren zur Herstellung einer Ribozym-Bibliothek gemäß Anspruch 1-3,
dadurch gekennzeichnet,
daß synthetische Oligonukleotide mit einer Zufallssequenz von 6-13 Nukleotiden und der Ribozymsequenz hergestellt, in einen Doppelstrang umgewandelt und über flankierende Restriktionsorte in die entsprechende Insertionsstelle der Expressionskassette gemäß den Ansprüchen 1 bis 2 kloniert werden.

5. Verwendung der gemäß Anspruch 4 erhaltenen Ribozym-Bibliothek
dadurch gekennzeichnet,
daß man das die gewünschte Zielsequenz enthaltende Material mit der Ribozym-Bibliothek inkubiert, die erhaltenen Spaltprodukte identifiziert und die für die Spaltung verantwortlichen Ribozyme isoliert.

6. Verwendung nach Anspruch 5,
dadurch gekennzeichnet,
daß als Material
- in vitro transkribierte RNS,
- Total-RNS oder
- zytoplasmatische RNS von Zellen
eingesetzt und die Inkubation in Anwesenheit von 100 µM Mg-Salz durchgeführt wird.

7. Verwendung nach Anspruch 5,
dadurch gekennzeichnet,
daß die Identifizierung der Spaltprodukte durch die PCR-Reaktion mit genspezifischen Primern und nachfolgende Gelelektrophorese erfolgt.

8. Verwendung nach Anspruch 5,
dadurch gekennzeichnet,
daß die für die Spaltung verantwortlichen Ribozyme durch Ausschneiden der Spaltprodukte aus dem Gel und nachfolgende Sequenzierung der Spaltstellen identifiziert werden.

9. Verwendung nach Anspruch 5,
dadurch gekennzeichnet,
daß das als wirksam erkannte Ribozym aus der Bibliothek durch Hybridisierung mit 2 für dieses Ribozym spezifischen Oligonukleotiden isoliert und als Expressionsklon verwendet wird.

## Claims

1. An expression cassette comprising a T7 promoter, an adenoviral va-RNA gene and a stable loop region with a ribozyme gene within the loop region, whereby the ribozyme gene comprises a central hammerhead structure, a defined sequence and flanking sequences on both sides of the hammerhead structure each with 6-13 nucleotides of random base sequence.

2. An expression cassette as claimed in Claim 1, characterised in that the central hammerhead structure includes a double-strand DNA of the sequence CTGATGAGTCCGTGAGGACGAAAC.

3. Use of the expression cassette as claimed in Claim 1 or 2 for constructing a ribozyme library having 10⁹-10¹¹ ribozyme genes.

4. A process for producing a ribozyme library as claimed in Claims 1 to 3, characterised in that synthetic oligonucleotides can be produced having a random sequence of 6-13 nucleotides and the ribozyme sequence. converted to a double strand and cloned by means of flanking restriction points into the corresponding insertion parts of the expression cassette as claimed in Claims I to 2.

5. Use of the resulting ribozyme library as claimed in Claim 4, characterised in that the material containing the desired target sequence is incubated with the ribozyme library, the resulting fission products are identified and the ribozyme responsible for the fission is isolated.

6. Use as claimed in Claim 5, characterised in that
- in vitro-transcribed RNS.
- total RNS or
- cytoplasmic RNS of cells is used as material and incubation is carried out in the presence of 100 µM Mg salt.

7. Use as claimed in Claim 5, characterised in that the fission products are identified by the PCR reaction with gene-specific primers and subsequent gel electrophoresis.

8. Use as claimed in Claim 5, characterised in that the ribozymes responsible for fission are identified by excision of the fission products from the gel and subsequent sequencing of the fission sites.

9. Use as claimed in Claim 5, characterised in that the ribozyme recognised as effective is isolated from the library by hybridising with 2 oligonucleotides specific to this ribozyme, and is used as expression clone.

## Revendications

1. Cassette d'expression composée d'un promoteur T7, d'un va-ARN-Gen adénoviral et d'une région de boucle stable contenant un gène de ribozyme, dans laquelle le gène de ribozyme se compose d'une structure centrale en T de séquence définie, et de séquences flanquantes de part et d'autre de la structure en T, comprenant chacune 6 à 13 nucléotides dans un ordre de bases quelconque.

2. Cassette d'expression selon la revendication 1, caractérisée en ce que la structure en T centrale comprend un ADN à double brin dont la séquence est CTGATGAGTCCGTGAGGACGAAAC.

3. Utilisation de la cassette d'expression selon la revendication 1 ou 2 pour la constitution d'une bibliothèque de ribozymes contenant 10⁹ à 10¹¹ gênes de ribozyme.

4. Procédé pour la constitution d'une bibliothèque de ribozymes selon les revendications 1 à 3, caractérisé en ce que des oligonucléotides de synthèse possédant une séquence aléatoire de 6 à 13 nucléotides et la séquence de ribozyme sont produits, transformés en un double brin et clonés à l'aide de sites de restriction flanquants dans la zone d'insertion correspondante de la cassette d'expression selon les revendications 1 à 2.

5. Utilisation de la bibliothèque de ribozymes constituée selon la revendication 4, caractérisée en ce que l'on fait incuber le matériel contenant la séquence cible souhaitée avec la bibliothèque de ribozymes, que l'on identifie les produits de dissociation obtenus et que l'on isole les ribozymes responsables de la dissociation.

6. Utilisation selon la revendication 5, caractérisée en ce que le matériel utilisé est
- de l'ARN transcrit *in vitro,*
- de l'ARN total ou
- de l'ARN de cytoplasme cellulaire
et l'incubation est effectuée en présence de 100 µM de sel de magnésium.

7. Utilisation selon la revendication 5, caractérisée en ce que l'identification des produits de dissociation est effectuée par ACP avec des amorces spécifiques des gènes puis électrophorèse sur gel.

8. Utilisation selon la revendication 5, caractérisée en ce que les ribozymes responsables de la dissociation sont identifiés par séparation des produits de dissociation sur le gel puis séquençage des points de dissociation.

9. Utilisation selon la revendication 5, caractérisée en ce que le ribozyme reconnu comme actif est isolé dans la bibliothèque par hybridation avec deux oligonucléotides spécifiques de ce ribozyme et utilisé comme clone d'expression.
